# NEW EUROPEAN PATENT SPECIFICATION

(11) **EP 2 124 573 B2**
(45) Date of publication and mention of the opposition decision: **15.05.2019**
(45) Mention of the grant of the patent: 04.05.2011
(21) Application number: 08709649.1
(22) Date of filing: 26.02.2008
(51) Int. Cl.: A01N 63/00, A01P 7/02, A01P 7/04, A01K 67/033

(54) **MITE COMPOSITION**
MILBENZUSAMMENSETZUNG
COMPOSITION D'ACARIENS

(30) Priority: 26.02.2007 GB 0703672; 26.05.2007 GB 0710122
(43) Date of publication of application: 02.12.2009
(73) Proprietor: Certis Europe B.V., 3605 MA Maarssen (NL)
(72) Inventor: SMYTHEMAN, Peter, Ronald, Ashford Kent TN24 0HY (GB)
(74) Representative: Williams, Andrea
(86) International application number: PCT/GB2008/050129
(87) International publication number: WO 2008/104807

(56) References cited:
- WO-A-2006/057552
- WO-A-2006/071107
- WO-A-2008/015393
- GILKESON L A: "MASS REARING OF PHYTOSEIID MITES FOR TESTING AND COMMERCIAL APPLICATION" ADVANCES IN INSECT REARING FOR RESEARCH AND PEST MANAGEMENT, XX, XX, 1 January 1992 (1992-01-01), pages 489-506, XP003002501
- ZHANG Y ET AL: "Potential of amblyseius cucumeris (Acari: phytoseiidae) as a biocontrol agent against schizotetranychus nanjingensis in Fujian, China" SYSTEMATIC AND APPLIED ACAROLOGY SPECIAL PUBLICATIONS, SYSTEMATIC AND APPLIED ACAROLOGY SOCIETY, LONDON, GB, vol. 4, 1 January 2000 (2000-01-01), pages 109-124, XP003002569 ISSN: 1461-0183
- BEGLYAROV ET AL: "The flour mite Acarus siro for mass breeding of phytoseiids (RU)" ZASHCHITA RASTENII, AGRORUS', MOSCOW, RU, no. 10, 1 January 1990 (1990-01-01), page 25, XP003014946
- RAMAKERS P M J: "Mass production and introduction of amlysieus mckenziei and a. cucumeris" IOBC WPRS BULLETIN, XX, XX, vol. 6, no. 3, 1 January 1983 (1983-01-01), pages 203-206, XP003000200 ISSN: 0253-1100
- KERS P J M ET AL: "Start of commercial production and introduction of amblyseius mckenziei sch. & pr. cacarina" DELINGEN VAN DE FACULTEIT LANDBOUWWETENSCHAPPEN UNIVERSITEITGENT, GENT, BE, vol. 47, no. 2, 1 January 1982 (1982-01-01), pages 541-545, XP003000199 ISSN: 0368-9697

## Description

### Technical Field

The present invention relates to a mite composition, a method for rearing a mite, a rearing system, a use of the composition in biological pest control, and to a method of biological pest control.

### Introduction

Biological pest control is a component of an integrated pest management strategy in agriculture and is an alternative to treating crops with insecticides. It is described as the reduction of pest populations by natural enemies. Pests that affect plants and such have an impact on crop yield include aphids, caterpillars, thrips, mites, white-flies and slugs. Many commercial insectaries rear and market a variety of natural enemies, including predaceous mites, lady beetles, lacewings, praying mantids, and several species of parasitoids. For example, it is known to use a composition of a rearing population of predatory mite fed on a factitious host supported on a carrier in biological pest control. Such a composition is often supplied in a housing having a predatory mite exit aperture. The housing is placed on or adjacent to plants and the predatory mite exits the housing to prey on unwanted pests such as white-flies, thrips, spider mites and tarsonemid mites. Examples of such compositions comprising a predatory mite and a host mite population from the family Acaridae are described in the literature, including WO2006057552 and WO2006071107 in the name of Koppert BV. One example is the predaceous mite *Phytoseiulus persimilis* which is used for control of the two-spotted spider mite. Another mite that has been used in pest control is *Amblyseius cucumeris,* a small predator that feeds on a wide range of small arthropod prey. It is used primarily for control of thrips, particularly *Frankliniella occidentalis,* but can also be used for control of Tarsonemid mites on a number of crops and can reduce Rust Mite (*Eriophyidae*) populations. Methods for isolating predatory mites from their natural environments and for culturing predatory mites are known in the art.

A problem for the manufacturers of mite compositions for use in biological pest control is finding an economical and easily bred host for predatory mites. Different mite families and indeed different mite species have different characteristics and requirements, such as breeding temperature, life cycle, size which results in different behaviour when using the mite to serve as a food source for a predatory mite. Other criteria which are important include the ability to live and thrive in an environment also suitable for the predatory mite, not to produce toxins which adversely affect the predatory mite, and to have a multiplication rate which does not lead to an overwhelming of the culture, but still is rapid enough to maintain a steady food supply for the predator which it supports, without its self being too rapidly eliminated. Furthermore, the impact of the mite on the crop plants should be minimal. Another factors to consider include the allergenic properties of the mite as this causes problems in production and in products which are shaken over the crop.

The inventors have found that a host comprising species from the family *Suidasiidae,* e.g. *Suidasia medanensis* (Oudemans, 1924) can be economically and easily bred. A benefit of *Suidasia medanensis* is that it is very small, and all life stages (adults, nymphs and eggs) of *Suidasia medanensis* are of a size which can be eaten by young and adult prey mites. Such a host has been found particularly useful for rearing phytoseiid predatory mite species, such as *Amblyseius swirskii.* In its natural habitat, *Amblyseius swirskii* feeds on plant feeding mites such as spider mites, tarsonemid and eriophyid mites and a range of plant dwelling insects. In nature, *Amblyseius swirskii* does not encounter mites from the family of *Suidasiidae* and *Chortoglyphidae* which are not plant dwelling species.

### Description of Invention

The invention relates to a mite composition comprising a breeding population of a predatory mite species selected from *Amblyseius swirskii,* and a factitious host population selected from the family of *Suidasiidae.*

The population of the predatory mite and the host population are each preferably breeding populations, thus leading to an increase in the number of individuals present in the population. The population comprises adults from both sexes and preferably individuals of other stages in the life cycle.

Mites as described herein are isolated from their natural environment and combined in the composition.

The host population comprises at least one species selected from the family *Suidasiidae.* The preferred genus is *Suidasia.* The host species may be selected from *Suidasia medanensis, S. medanensis, S. nesbitti* , *S. pontifica* or *S. reticulata.* The most preferred host species of the family *Suidasiidae* is *Suidasia medanensis* (Oudemans, 1924).

In one embodiment, the host population may comprise a mixture of at least two different species, for example from the same genus or from different genera. For example, the composition may comprise a combination of *Acarus gracilis, Suidasia medanensis and Chortoglyphus arcuatus.* Furthermore, *Suidasia medanensis* may also be combined with other known species, such as *Carpoglyphus.*

The breeding population of predatory mite species is the phytoseiid predatory mite species *Amblyseius swirskii,.*

Thus, in one preferred aspect, the invention relates to a composition comprising a population of *Amblyseius swirskii* and a host population of *Suidasia medanensis.*

Preferably, the composition comprises a carrier for individuals of said populations. The carrier may be bran flakes or wood chips or other similar organic materials, vermiculite or any other suitable light granular material. The composition may further comprise a food substance suitable for the factitious host population. The composition may further comprise an inert component such as vermiculite. Preferably, the number of individuals of the predatory mite species relative to the number of individuals of the factitious host is from 100:1 to 1:20, such as 1:1 to 1: 10, e.g. 1:4, 1:5 or 1: 7. A skilled person would know that these amounts represent a general guide and that the exact dosage depends on the species used. As this is a living culture the precise ratios will vary over time as the food supply and prey species is consumed and the predator population multiplies. However, in general, the number of individuals of the host population should exceed that of the predatory mite.

According to the invention, the composition described herein can be used in a method for biological crop protection and biological pest control. The use of the composition is particularly beneficial because it allows mass rearing of *Amblyseius swirskii.* The host population in the composition is a factitious host and serves as a source of food for the predatory mite if other food sources are not available. The composition and methods described herein can be used on crops such as vegetables, fruit crops, ornamental crops or tree crops, e.g. peppers, eggplants, cucumbers, melons, watermelons, strawberries, raspberries, roses, gerberas, chrysanthemums, or citrus trees or any other suitable crops. The pest may be selected from white-flies, thrips, spider mites and tarsonemid mites.

According to another aspect of the present invention there is provided a method for rearing or culturing a predatory mite such as *Amblyseius swirskii,* comprising providing a composition according to the invention and allowing individuals of the predatory mite to prey on individuals of said factitious population. The method may further comprise maintaining the composition at 17-36°C and/or 59-96% humidity, e.g. 27°C and 85% humidity. The composition may comprise a carrier and a suitable food substance.

Another aspect of the present invention relates to use of the family *Suidasiidae* preferably of the genus *Suidasia,* such as *Suidasia medanensis,* as a factitious host for rearing the predatory mite *Amblyseius swirskii.*

Another aspect of the present invention relates to a method of biological pest control in a crop in which a composition as described herein is applied to a crop. The crop pest may be selected from white-flies, thrips, spider mites and tarsonemid mites. A suitable amount of the composition is applied, preferably to each crop plant. The amount may be from 1-15 ml preferably 2-7 ml. The composition may be applied to the crop in different ways, for example by sprinkling the crop with the composition or as the device as described herein.

Further modifications will be apparent to those skilled in the art without departing from the scope of the present invention.

Non limiting examples of the invention and suitability of Suidasiidae, e.g. *Suidasia medanensis* as a factitious host for rearing a predatory mite are given below.

### Examples

### Example 1

As indicated in the study data below, trials have shown that at 29°C and 75% RH the mean number of eggs laid per female *Amblyseius swirskii* is in the range 1.80 to 1.85 eggs per female per day when feeding on excess supply of *Suidasia medanensis.* This is a rate of reproduction which is very suitable for economic production of *Amblyseius swirskii.*

### Study data

Each replicate = 1 adult female *A.swirskii* on a leaf disc arena with all stages of *Suidasia medanensis* as food.

Number of offspring was counted after 48 hours.

Arenas were maintained at 29 deg.C and 75%RH

| Replicate | Ova | Nymph | Total | Daily mean |
|---|---|---|---|---|
| 1 | 3 | 1 | 4 | 2.00 |
| 2 | 3 | 1 | 4 | 2.00 |
| 3 | 2 | 0 | 2 | 1.00 |
| 4 | 4 | 0 | 4 | 2.00 |
| 5 | 1 | 0 | 1 | 0.50 |
| 6 | 3 | 0 | 3 | 1.50 |
| 7 | 3 | 1 | 4 | 2.00 |
| 8 | 4 | 1 | 5 | 2.50 |
| 9 | 4 | 1 | 5 | 2.50 |
| 10 | 5 | 0 | 5 | 2.50 |
| Total | 32 | 5 | 37 | |
| | | 48 hr mean | 3.70 | eggs/female/day |
| | | Daily mean | 1.85 | eggs/female/day |

Each replicate = 1 adult female *A.swirskii* on a leaf disc arena with all stages of *Suidasia medanensis* as food.

Number of offspring was counted after 72 hours.

Arenas were maintained at 29 deg.C and 75%RH

| Replicate | Ova | Nymph | Total | Daily mean |
|---|---|---|---|---|
| 1 | 1 | 3 | 4 | 1.33 |
| 2 | 3 | 1 | 4 | 1.33 |
| 3 | 1 | 3 | 4 | 1.33 |
| 4 | 5 | 3 | 8 | 2.67 |
| 5 | 4 | 1 | 5 | 1.67 |
| 6 | 2 | 2 | 4 | 1.33 |
| 7 | 5 | 1 | 6 | 2.00 |
| 8 | 1 | 2 | 3 | 1.00 |
| 9 | 3 | 4 | 7 | 2.33 |
| 10 | 2 | 2 | 4 | 1.33 |
| 11 | 2 | 2 | 4 | 1.33 |
| 12 | 7 | 2 | 9 | 3.00 |
| 13 | 5 | 2 | 7 | 2.33 |
| 14 | 6 | 0 | 6 | 2.00 |
| 15 | 4 | 2 | 6 | 2.00 |
| 16 | 4 | 2 | 6 | 2.00 |
| 17 | 3 | 3 | 6 | 2.00 |
| 18 | 5 | 2 | 7 | 2.33 |
| 19 | 3 | 3 | 6 | 2.00 |
| 20 | 3 | 2 | 5 | 1.67 |
| 21 | 2 | 4 | 6 | 2.00 |
| 22 | 2 | 4 | 6 | 2.00 |
| 23 | 2 | 1 | 3 | 1.00 |
| 24 | 3 | 1 | 4 | 1.33 |
| 25 | 6 | 0 | 6 | 2.00 |
| Total | 84 | 52 | 136 | |
| | | 72 hr mean | 5.44 | eggs/female/day |
| | | Daily mean | 1.81 | eggs/female/day |

### Example 2

A composition comprising a breeding population of predatory mite species *Amblyseius swirskii* and a factitious host comprising *Suidasia medanensis* are supported on a carrier such as bran flakes or wood chips, along with suitable foodstuffs such as cereal extracts, yeast etc. The composition is placed in a sachet with a predatory mite exit aperture. The sachet has a hook to hang the sachet on a plant. Preferably the number of individuals of the predatory might species relative to the number of individuals of the factitious host is from 100:1 to 1:20, such as 1:1 to 1: 10, e.g. 1:4, 1:5 or 1:7. The composition has proved useful in controlling white-flies, thrips, spider mites and tarsonemid mites on crops.

### Example 3

A composition comprising a breeding population of predatory mite species *Amblyseius swirskii* and a factitious host comprising *Suidasia medanensis* are supported on a carrier such as bran flakes or wood chips, along with suitable foodstuffs such as cereal extracts, yeasts etc. The mixture is diluted by adding extra carrier material such as bran. The number of individuals of the predatory mite species relative to the number of individuals of the factitious host may be from 100:1 to 1:20, such as 1:1 to 1: 10, e.g. 1:4, 1:5 or 1: 7. The composition may be added to a container such as a tube or bottle, from which the composition can be sprinkled or otherwise distributed onto a crop. The composition has proved useful in controlling white-flies, thrips, spider mites and tarsonemid mites on crops. *Suidasia medanensis* thrives in aerated media, and benefits from addition of a suitable inert component to the culture medium such as vermiculite. This also enhances the suitability of the culture environment for the production of plant and soil dwelling predatory species of mites. This is equally true in bulk commercial culture and in individual breeding sachets. Optimum culture conditions are around 27°C and 85% relative humidity.

## Claims

1. A composition comprising a population of a predatory mite selected from *Amblyseius swirskii* and a host mite population selected from the family of *Suidasiidae.*

2. A composition according to claim 1 wherein the host population is selected from *Suidasia.*

3. A composition according to claim 2 wherein the host population is *Suidasia medanensis.*

4. A composition according to a preceding claim wherein the host population is selected from the family of *Suidasiidae* and further comprises at least a second mite species.

5. A composition according to a preceding claim wherein the composition further comprises a carrier.

6. A method for biological pest control comprising applying a composition as defined in any of claims 1 to 5 to a crop.

7. The use of a composition as defined in any of claims 1 to 5 for biological pest control.

8. A method for rearing or culturing a population of a predatory mite comprising providing a host population of *Suidasia medanensis* to a predatory mite selected from *Amblyseius swirskii.*

## Patentansprüche

1. Zusammensetzung, umfassend eine Population einer Raubmilbe, ausgewählt aus *Amblyseius swirskii* und eine Wirtsmilbenpopulation, ausgewählt aus der Familie der *Suidasiidae.*

2. Zusammensetzung nach Anspruch 1, wobei die Wirtspopulation aus *Suidasia* ausgewählt ist.

3. Zusammensetzung nach Anspruch 2, wobei die Wirtspopulation *Suidasia medanensis* ist.

4. Zusammensetzung nach einem vorhergehenden Anspruch, wobei die Wirtspopulation aus der Familie der *Suidasiidae* ausgewählt ist und ferner mindestens eine zweite Milbenspezies umfasst.

5. Zusammensetzung nach einem vorhergehenden Anspruch, wobei die Zusammensetzung ferner einen Träger umfasst.

6. Verfahren zur biologischen Schädlingsbekämpfung, umfassend das Aufbringen einer Zusammensetzung, wie in einem der Ansprüche 1 bis 5 definiert, auf eine Feldfrucht.

7. Verwendung einer Zusammensetzung, wie in einem der Ansprüche 1 bis 5 definiert, zur biologischen Schädlingsbekämpfung.

8. Verfahren zum Züchten oder Kultivieren einer Population einer Raubmilbe, umfassend das Bereitstellen einer Wirtspopulation von *Suidasia medanensis* für eine Raubmilbe, ausgewählt aus *Amblyseius swirskii.*

## Revendications

1. Composition comprenant une population d'un acarien prédateur sélectionné parmi *Amblyseius swirskii,* et une population d'acariens hôtes sélectionnée dans la famille des *Suidasiidae.*

2. Composition selon la revendication 1, où la population hôte est sélectionnée parmi *Suidasia.*

3. Composition selon la revendication 2, où la population hôte est *Suidasia medanensis.*

4. Composition selon l'une quelconque des revendications précédentes où la population hôte est sélectionnée dans la famille des *Suidasiidae* et comprend également au moins une seconde espèce d'acarien.

5. Composition selon l'une quelconque des revendications précédentes où la composition comprend également un support.

6. Procédé de lutte antiparasitaire biologique qui comprend l'application d'une composition telle que définie dans l'une quelconque des revendications 1 à 5 sur une culture.

7. Utilisation d'une composition telle que définie dans l'une quelconque des revendications 1 à 5 pour la lutte antiparasitaire biologique.

8. Procédé d'élevage ou de culture d'une population d'un acarien prédateur qui consiste à fournir une population hôte de *Suidasia medanensis* à un acarien prédateur sélectionné parmi *Amblyseius swirskii.*
